# EUROPÄISCHE PATENTSCHRIFT

(11) **EP 1 778 622 B1**
(45) Veröffentlichungstag und Bekanntmachung des Hinweises auf die Patenterteilung: **13.01.2010**
(21) Anmeldenummer: 05777875.5
(22) Anmeldetag: 08.08.2005
(51) Int. Cl.: C07C 233/22, A61K 31/165, A61P 29/00

(54) **PARA-ALKYL-SUBSTITUIERTE N-(4-HYDROXY-3-METHOXY-BENZYL)-ZIMTSÄEUREAMIDE UND DEREN VERWENDUNG ZUR HERSTELLUNG VON ARZNEIMITTELN**
PARA-ALKYL-SUBSTITUTED N-(4-HYDROXY-3-METHOXY-BENZYL) CINNAMAMIDES AND THE USE THEREOF FOR PRODUCING DRUGS
N-(4-HYDROXY-3-METHOXY-BENZYL)-AMIDES D'ACIDE CINNAMIQUE A SUBSTITUTION PARA-ALKYLE ET LEUR UTILISATION POUR LA FABRICATION DE MEDICAMENTS

(30) Priorität: 12.08.2004 DE 102004039373
(43) Veröffentlichungstag der Anmeldung: 02.05.2007
(73) Patentinhaber: Grünenthal GmbH, 52078 Aachen (DE)
(72) Erfinder: KÜHNERT, Sven, 52355 Düren (DE); FRANK, Robert, 52070 Aachen (DE); JOSTOCK, Ruth, 52223 Stolberg (DE)
(74) Vertreter: Brosch, Oliver
(86) Internationale Anmeldenummer: PCT/EP2005/053898
(87) Internationale Veröffentlichungsnummer: WO 2006/018406

(56) Entgegenhaltungen:
- C. S. J. WALPOLE ET AL.: "Analogues of Capsaicin with Agonist Activity as Novel Analgesic Agents; Structure-Activity Studies. 3. The Hydrophobic Side-Chain "C-Region"" J MED CHEMISTRY, Bd. 36, 1993, Seiten 2381-2389, XP002354678
- T. W.SCHULTZ, T. S. RANNEY: "Structure-Activity Relationships for Osteolathyrism: II. Effects of Alkyl-Substituted Acid Hydrazides" TOXICOLOGY, Bd. 53, 1988, Seiten 147-159, XP002354679

## Beschreibung

Die vorliegende Erfindung betrifft *para-*Alkyl-substituierte N-(4-Hydroxy-3-methoxybenzyl)-zimtsäursamide. Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände,
wobei hierunter die erfolgreiche und zufriedenstellende Schmersbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz; insbesondere von Schmerz ausgewählt aus der Gruppe bestehend aus akutem Schmerz, chronischem Schmerz, neuropathischem Schmerz und visceralem Schmerz, besonders bevorzugt von neurophatischem Schmerz; stellt der Vanilloid-Rezeptor vom Subtyp 1 (VR1/TRPV1) dar, der häufig auch als Capsaicin-Rezeptor bezeichnet wird. Dieser Rezeptor wird u.a durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus: Erkrankungen motorischer Neurone: Störungen des kardiovaskulären Systems: Störungen der Nahrungsaufnahme; Medikamentenabhängigkeit; Medikamentenmißbrauch und insbesondere Haminkontinzenz.

Walpole et al., J. Med. Chem. 1993 (36) 2381-2389 offenbart Capsaicin-Analoga mit analgetischer Wirkung. Der Veröffentlichung von Schultz und Ranney, Toxicology, 1988 (53) 147-159) sind Untersuchungen zu Struktur-Wirkungs-Beziehungen bei Alkyl-substituierten Säurehydraziden zu entnehmen.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, daß die *para-*Alkyl-substituierten N-(4-Hydroxy-3-methoxy-benzyl)-zimtsäureamide der nachstehend angegebenen allgemeinen Formeln I und Ia eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1 /TRPV1) vermittelt werden.

Bevorzugt eignen sich die erfindungsgemäßen Verbindungen zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt Gedächtnisstörungen; Angstzuständen; Epilepsie; Atemwegserkrankungen, vorzugsweise ausgewählt aus der Gruppe bestehend aus Asthma und Lungenentzündungen; Husten; Harninkontinenz; einer überaktiven Blase (overactive bladder, OAB); Diarrhöe; Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmißbrauch; Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur modulation der Bewegungsaktivität oder zur Lokalanästhesie.

Ein Gegenstand der vorliegenden Erfindung sind daher *para*-Alkyl-substituierte N-(4-Hydroxy-3-methoxy-benzyl)-zimtsäureamide der allgemeinen Formel I, 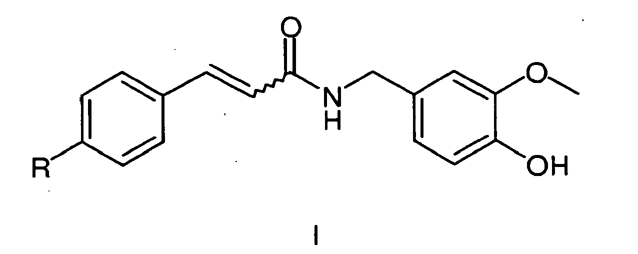 worin
R für einen linearen oder verzweigten Alkyl-Rest steht,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Der Fachmann versteht, daß in den erfindungsgemäßen Verbindungen der allgemeinen Formel I die von Wasserstoff verschiedenen Substituenten an der Doppelbindung der *para*-Alkyl-substituierten N-(4-Hydroxy-3-methoxy-benzyl)-zimtsäureamide sowohl *cis* als auch *trans* zueinander angeordnet sein können. Das entsprechende *trans-*Isomer wird häufig auch als (*E*)-Isomer, das *cis*-Isomer als (*Z*)-Isomer bezeichnet.

Bevorzugt sind *para*-Alkyl-substituierte *trans*-N-(4-Hydroxy-3-methoxy-benzyl)-zimtsäureamide der allgemeinen Formel la, 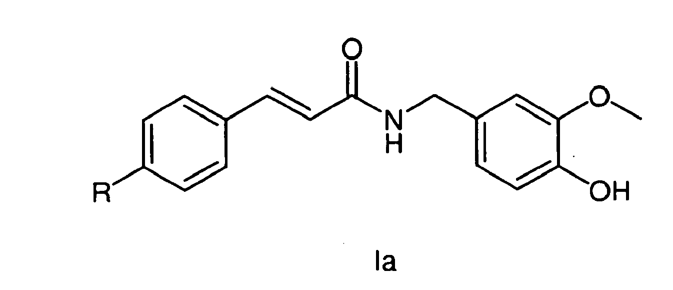 worin
R für einen linearen oder verzweigten Alkyl-Rest steht,
jeweils ggf. in Form eines ihrer reinen Stereoisomeren, insbesondere Enantiomeren, ihrer Racemate oder in Form einer Mischung von Enantiomeren, in einem beliebigen Mischungsverhältnis, oder jeweils in Form entsprechender Salze, oder jeweils in Form entsprechender Solvate.

Bevorzugt sind auch jeweils Verbindungen der allgemeinen Formeln I und Ia, in denen R für einen linearen oder verzweigten C₁₋₂₀-Alkyl-Rest steht,
besonders bevorzugt für einen linearen oder verzweigten C₁₋₁₀-Alkyl-Rest steht,
ganz besonders bevorzugt für einen Alkyl-Rest ausgewählt aus der Gruppe bestehend aus Methyl; Ethyl; n-Propyl; iso-Propyl; n-Butyl; iso-Butyl; sec-Butyl; tert-Butyl; n-Pentyl; 2-Pentyl; 3-Pentyl; iso-Pentyl; neo-Pentyl; 1,1-Dimethylpropyl; 1,2-Dimethylpropyl; n-Hexyl; 2-Hexyl; 3-Hexyl; iso-Hexyl; neo-Hexyl; n-Heptyl; 2-Heptyl; 3-Heptyl; 4-Heptyl; iso-Heptyl; neo-Heptyl; n-Octyl; 2-Octyl; 3-Octyl; 4-Octyl; isoOctyl; neo-Octyl; n-Nonyl; 2-Nonyl; 3-Nonyl; 4-Nonyl; 5-Nonyl; iso-Nonyl; neo-Nonyl und n-Decyl steht,

im noch weiter bevorzugten Sinn für einen Alkyl-Rest ausgewählt aus der Gruppe bestehend aus Methyl; Ethyl; n-Propyl; iso-Propyl; n-Butyl; iso-Butyl; tert-Butyl; neo-Pentyl und n-Octyl steht.

Besonders bevorzugt sind die Verbindungen der allgemeinen Formeln I und Ia ausgewählt aus der Gruppe bestehend aus
[1] *para*-Methyl-*trans*-N-(4-hydroxy-3-methoxy-benzyl)-zimtsäureamid,
[2] *para*-Ethyl-*trans*-N-(4-hydroxy-3-methoxy-benzyl)-zimtsäureamid,
[3] *para*-iso-Propyl-trans-N-(4-hydroxy-3-methoxy-benzyl)-zimtsäureamid,
[4] *para*-tert-Butyl-*trans*-N-(4-hydroxy-3-methoxy-benzyl)-zimtsäureamid,
[5] *para*-Propyl-trans-N-(4-hydroxy-3-methoxy-benzyl)-zimtsäureamid,
[6] *para*-iso-Butyl-trans-N-(4-hydroxy-3-methoxy-benzyl)-zimtsäureamid,
[7] *para*-neo-Pentyl-trans-N-(4-hydroxy-3-methoxy-benzyl)-zimtsäureamid,
[8] *para*-Butyl-trans-N-(4-hydroxy-3-methoxy-benzyl)-zimtsäureamid und
[9] *para*-Octyl-trans-N-(4-hydroxy-3-methoxy-benzyl)-zimtsäureamid;
   und jeweils deren Salze, jeweils ggf. in Form entsprechender Solvate.

Ganz besonders bevorzugt ist die Verbindung

### [4] para-tert-Butyl-trans-N-(4-hydroxy-3-methoxy-benzyl)-zimtsäureamid;

und deren Salze, jeweils ggf. in Form entsprechender Solvate.

Sofern die erfindungsgemäßen Verbindungen der allgemeinen Formeln I und Ia in Form ihrer Salze vorliegen, können diese bevorzugt ausgewählt werden aus der Gruppe der Alkalimetallsalze, vorzugsweise der Natrium- oder Kaliumsalze. Ebenfalls bevorzugt sind Salze mit Kationen der allgemeinen Formel [NRₓH₄₋ₓ]⁺, worin R für einen linearen oder ggf. verzweigten Alkylrest mit 1 bis 4 Kohlenstoffatomen und x für 0, 1, 2, 3 oder 4 steht. Die vorstehend genannten Salze können auch jeweils in Form entsprechender Solvate, vorzugsweise in Form der Hydrate, vorliegen.

Ein weiterer Gegenstand der vorliegenden Erfindung ist ein Verfahren zur Herstellung der erfindungsgemäßen Verbindungen der vorstehend angegebenen allgemeinen Formeln I und Ia gemäß dem wenigstens ein Aldehyd der allgemeinen Formel II, 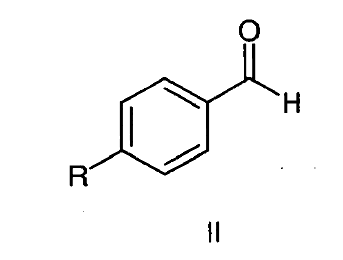 worin R die vorstehend genannte Bedeutung hat, mit Malonsäure (OH-C(=O)-CH₂-C(=O)-OH) ggf. in einem Reaktionsmedium, in Gegenwart wenigstens einer Base umgesetzt wird, die so erhaltene *para-*Alkyl substituierte Zimtsäure der allgemeinen Formel III, 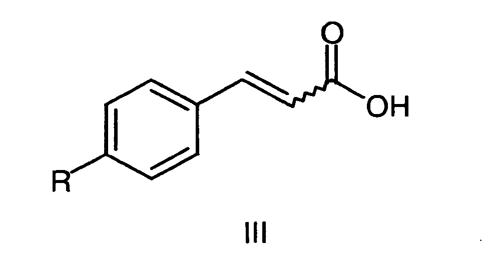 worin R die vorstehend genannte Bedeutung hat, ggf. in Form eines entsprechenden Salzes ggf. isoliert und ggf. gereinigt wird, und durch Umsetzung mit 4-Hydroxy-3-methoxybenzylamin, ggf. in Form eines entsprechenden Salzes, vorzugsweise in Form des Hydrochlorids, in einem Reaktionsmedium, ggf. in Gegenwart wenigstens einer Base, ggf. in Gegenwart wenigstens eines geeigneten Kopplungsmittels, in eine entsprechende Verbindung der allgemeinen Formel I, 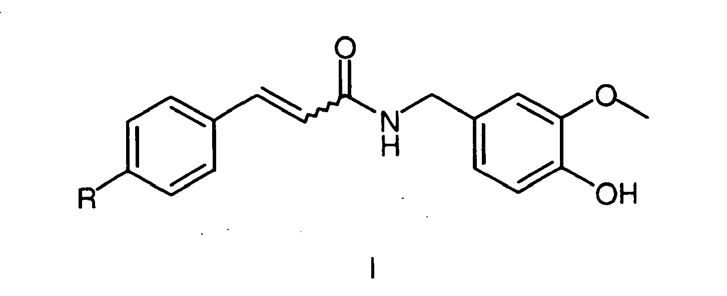 ggf. in Form eines entsprechenden Salzes, überführt wird worin R die vorstehend genannte Bedeutung hat, und diese ggf. gereinigt und ggf. isoliert wird.

Das erfindungsgemäße Verfahren zur Herstellung *para*-Alkyl-substituierter N-(4-Hydroxy-3-methoxy-benzyl)-zimtsäureamide ist auch im folgenden Schema 1 angegeben.

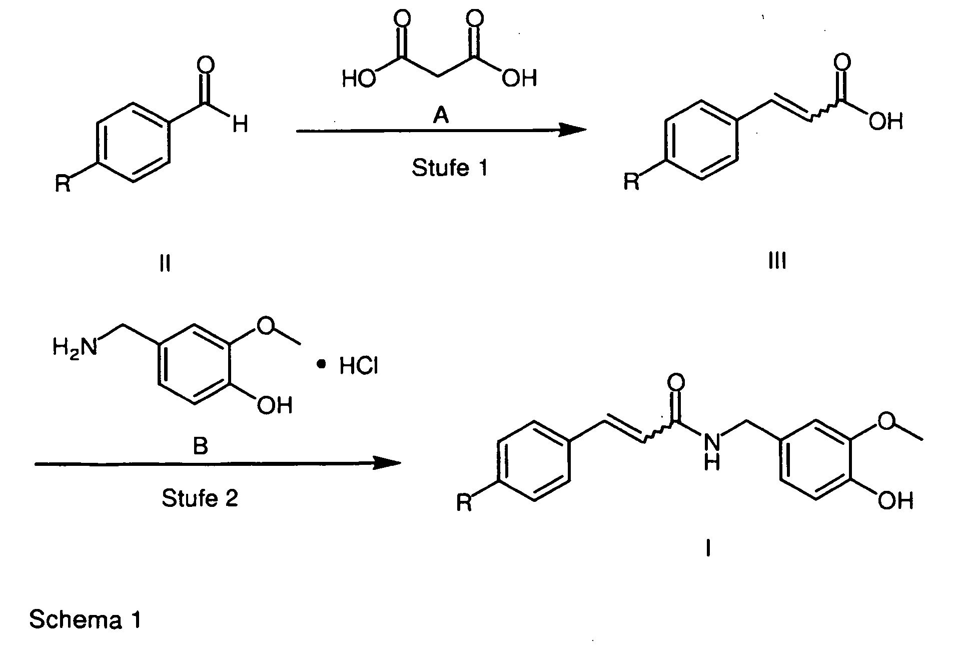

In Stufe 1 werden Aldehyde der vorstehend angegebenen Formel II, worin R die vorstehend genannte Bedeutung hat, mit Malonsäure (Verbindung A), ggf. in einem geeigneten Reaktionsmedium, vorzugsweise ausgewählt aus der Gruppe bestehend aus Tetrahydrofuran, Dimethylformamid, Dimethylacetamid, Acetonitril, Pyridin, Dimethylsulfoxid, Xylol, Toluol und Mischungen aus wenigstens zwei der vorstehend genannten Reaktionsmedien, ggf. in Gegenwart wenigstens einer Base, vorzugsweise einer organischen Base ausgewählt aus der Gruppe bestehend aus Piperidin, Pyridin, Dimethylaminopyridin, Triethylamin und Diisopropylethylamin vorzugsweise bei Temperaturen von 20 °C bis 150 °C, besonders bevorzugt bei einer Temperatur von 60 °C bis 120 °C, zu para-Alkyl substituierten Zimtsäuren der allgemeinen Formel III, worin R die vorstehend genannte Bedeutung hat, ggf. in Form eines entsprechenden Salzes, umgesetzt und diese ggf. isoliert und ggf. gereinigt. Besonders bevorzugt erfolgt die Umsetzung in Pyridin in Gegenwart von Piperidin.

In Stufe 2 werden Verbindungen der vorstehend angegebenen allgemeinen Formel III mit 4-Hydroxy-3-methoxybenzylamin, ggf. in Form eines entsprechenden Salzes, vorzugsweise in Form des Hydrochlorids (Verbindung B), in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und einer Mischung aus wenigstens zwei der vorstehend genannten Reaktionsmedien, ggf. in Gegenwart wenigstens eines geeigneten Kopplungsmittels, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), 1,1'-Carbonyldiimidazol (CDI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluronium-tetrafluoroborat (TBTU) und 1-Hydroxy-7-azabenzotriazol (HOAt), ggf. in Gegenwart wenigstens einer organischen Base, vorzugsweise ausgewählt aus der Gruppe bestehend aus Triethylamin, Piperidin, N-Methylmorpholin, Pyridin, Dimethylaminopyridin und Diisopropylethylamin vorzugsweise bei Temperaturen von -70°C bis 100°C zu Verbindungen der allgemeinen Formel I, ggf. in Form eines entsprechenden Salzes, vorzugsweise in Form des entsprechenden Hydrochlorids, umgesetzt, und diese ggf. gereinigt und ggf. isoliert.Die nach dem erfindungsgemäßen Verfahren erhaltene *para-*Alkyl substituierte Zimtsäure der allgemeinen Formel III liegt üblicherweise als Gemisch ihrer *cis*/*trans*-Isomere vor, aus dem nach üblichen, dem Fachmann bekannten Methoden das jeweilige Diastereomer, insbesondere das *trans*-Isomer der allgemeinen Formel IIIa, 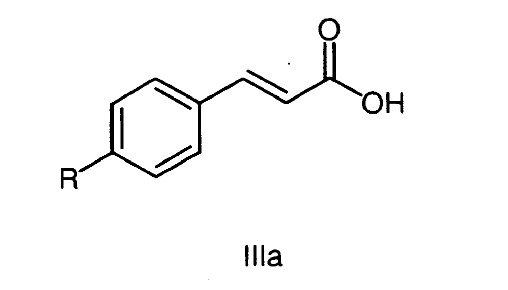 isoliert und ggf. gereinigt werden kann. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, oder die Umkristallisation aus einem geeigneten Lösungsmittel, vorzugsweise aus Methanol, genannt. Die *para*-Alkyl substituierte Zimtsäure der allgemeinen Formel IIIa kann ebenfalls in Stufe 2 des erfindungsgemäßen Verfahrens gemäß Schema 1 eingesetzt werden.

Sofern die nach dem erfindungsgemäßen Verfahren erhaltenen *para-*Alkyl-substituierten N-(4-Hydroxy-3-methoxy-benzyl)-zimtsäureamide als Gemisch ihrer *cis*/*trans*-Isomere vorliegen, kann daraus nach üblichen, dem Fachmann bekannten Methoden das *trans*-Isomer der allgemeinen Formel Ia, 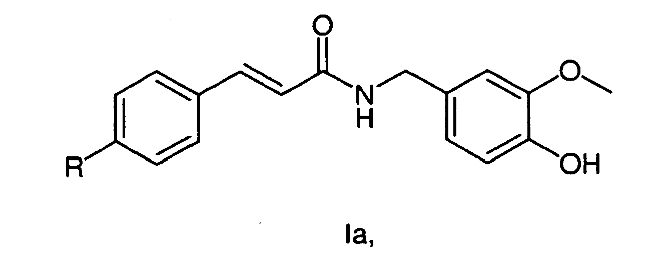 isoliert und ggf. gereinigt werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, oder die Umkristallisation aus einem geeigneten Lösungsmittel genannt.
Die Verbindungen der vorstehend angegebenen Formel II sowie die Verbindungen A und B, sind jeweils am Markt käuflich erhältlich und/oder können nach den üblichen, dem Fachmann bekannten Verfahren hergestellt werden.

Die vorstehend beschriebenen Umsetzungen können jeweils unter den üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Sofern die erfindungsgemäßen *para-*Alkyl-substituierten N-(4-Hydroxy-3-methoxybenzyl)-zimtsäureamide der vorstehend angegebenen allgemeinen Formeln I und Ia nach ihrer Herstellung in Form einer Mischung ihrer Enantiomeren oder Diastereomeren erhalten werden, können diese nach üblichen, dem Fachmann bekannten Verfahren getrennt und ggf. isoliert werden. Beispielhaft seien chromatographische Trennverfahren an chiraler Phase, insbesondere Flüssigkeits-chromatographie-Verfahren unter Normaldruck oder unter erhöhtem Druck, bevorzugt MPLC- und HPLC-Verfahren genannt.

Die erfindungsgemäßen *para-*Alkyl-substituierten N-(4-Hydroxy-3-methoxy-benzyl)-zimtsäureamide der vorstehend angegebenen allgemeinen Formeln I und Ia sowie ggf. entsprechende Stereoisomere können nach den üblichen, dem Fachmann bekannten Verfahren in Form entsprechender Salze, insbesondere in Form entsprechender physiologisch verträglicher Salze erhalten werden, wobei das erfindungsgemäße Arzneimittel eines oder mehrere Salze einer oder mehrerer dieser Verbindungen aufweisen kann.

Die erfindungsgemäßen *para-*Alkyl-substituierten N-(4-Hydroxy-3-methoxy-benzyl)-zimtsäureamide der vorstehend angegebenen allgemeinen Formeln I und Ia sowie ggf. entsprechende Stereoisomere und jeweils deren physiologisch verträgliche Salze können nach üblichen, dem Fachmann bekannten Verfahren auch in Form ihrer Solvate, insbesondere in Form ihrer Hydrate erhalten werden.

Es wurde überraschenderweise gefunden, daß sich die erfindungsgemäßen *para-*Alkyl-substituierten N-(4-Hydroxy-3-methoxy-benzyl)-zimtsäureamide der vorstehend angegebenen allgemeinen Formeln I und Ia zur Vanilloid-Rezeptor 1-(VR1/TRPV1)-Regulation, vorzugsweise als Agonisten zur Vanilloid-Rezeptor 1-(VR1/TRPV1)-Aktivierung, eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Die erfindungsgemäßen *para-*Alkyl-substituierten N-(4-Hydroxy-3-methoxy-benzyl)-zimtsäureamide der vorstehend angegebenen allgemeinen Formeln I und Ia und ggf. entsprechende Stereoisomere sowie jeweils die entsprechenden Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens ein erfindungsgemäßes *para-*Alkyl-substituiertes N-(4-Hydroxy-3-methoxy-benzyl)-zimtsäureamid der vorstehend angegebenen allgemeinen Formel I bzw. Ia, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate sowie ggf. einen oder mehrere pharmazeutisch verträgliche Hilfsstoffe.

Das erfindungsgemäße Arzneimittel eignet sich zur Vanilloid-Rezeptor 1-(VR1/TRPV1)-Regulation, vorzugsweise zur Vanilloid-Rezeptor 1-(VR1/TRPV1)-Aktivierung. Bevorzugt eignet sich das erfindungsgemäße Arzneimittel daher zur Prophylaxe und/oder Behandlung von Störungen und/oder Krankheiten, die zumindest teilweise durch Vanilloid-Rezeptoren 1 (VR1/TRPV1) vermittelt werden.

Bevorzugt eignet sich das erfindungsgemäße Arzneimittel zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, besonders bevorzugt ausgewählt aus der Gruppe bestehend aus Multipler Sklerose, Morbus Alzheimer, Morbus Parkinson und Morbus Huntington; kognitiven Erkrankungen, besonders bevorzugt kognitiven Mangelzuständen, ganz besonders bevorzugt Gedächtnisstörungen; Angstzuständen; Epilepsie; Atemwegserkrankungen, vorzugsweise ausgewählt aus der Gruppe bestehend aus Asthma und Lungenentzündungen; Husten; Harninkontinenz; einer überaktiven Blase (overactive bladder, OAB); Diarrhöe; Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Pruritus; Störungen der Nahrungsaufnahme, besonders bevorzugt ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, besonders bevorzugt gegenüber natürlichen oder synthetischen Opioiden; Drogenabhängigkeit; Drogenmißbrauch; Entzugserscheinungen bei Drogenabhängigkeit; Alkoholabhängigkeit; Alkoholmißbrauch; Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

Besonders geeignet ist das erfindungsgemäße Arzneimittel zur Prophylaxe und/oder Behandlung von Harninkontinenz oder von Schmerz, insbesondere von Schmerz ausgewählt aus der Gruppe bestehend aus akutem Schmerz, chronischem Schmerz, neuropathischem Schmerz und visceralem Schmerz.

Ganz besonders geeignet ist das erfindungsgemäße Arzneimittel zur Prophylaxe und/oder Behandlung von Schmerz, insbesondere von Schmerz ausgewählt aus der Gruppe bestehend aus akutem Schmerz, chronischem Schmerz, neuropathischem Schmerz und visceralem Schmerz.

Ein weiterer Gegenstand der vorliegenden Erfindung ist die Verwendung wenigstens eines erfindungsgemäßen *para-*Alkyl-substituierten N-(4-Hydroxy-3-methoxy-benzyl)-zimtsäureamids der vorstehend angegebenen allgemeinen Formel I bzw. Ia, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate; sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Aktivierung.

Bevorzugt ist die Verwendung wenigstens eines erfindungsgemäßen *para-*Alkyl-substituierten N-(4-Hydroxy-3-methoxy-benzyl)-zimtsäureamids der vorstehend angegebenen allgemeinen Formel I bzw. Ia, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate; sowie ggf. eines oder mehrerer pharmazeutisch verträglicher Hilfsstoffe zur Herstellung eines Arzneimittels zur Prophylaxe und/oder Behandlung von Störungen und/oder Krankheiten, die zumindest teilweise durch Vanilloid-Rezeptoren 1 (VR1/TRPV1) vermittelt werden.

Bevorzugt ist insbesondere auch die Verwendung wenigstens eines erfindungsgemäßen *para-*Alkyl-substituierten N-(4-Hydroxy-3-methoxy-benzyl)-zimtsäureamids der vorstehend angegebenen allgemeinen Formel I bzw. Ia, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate sowie ggf. eines oder mehrerer pharmazeutisch verträglicher Hilfsstoffe zur Herstellung eines Arzneimittels zur Prophylaxe und/oder Behandlung von Schmerz, besonders bevorzugt von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, besonders bevorrzugt ausgewählt aus der Gruppe bestehend aus Multipler Sklerose, Morbus Alzheimer, Morbus Parkinson und Morbus Huntington; kognitiven Erkrankungen, besonders bevorzugt kognitiven Mangelzuständen, ganz besonders bevorzugt Gedächtnisstörungen; Angstzuständen; Epilepsie; Atemwegserkrankungen, vorzugsweise ausgewählt aus der Gruppe bestehend aus Asthma und Lungenentzündungen; Husten; Harninkontinenz; einer überaktiven Blase (overactive bladder, OAB); Diarrhöe; Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Pruritus; Störungen der Nahrungsaufnahme, besonders bevorzugt ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmißbrauch; Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie, vorzugsweise zur Prophylaxe und/oder Behandlung von Harninkontinenz oder Schmerz, vorzugsweise von Schmerz ausgewählt aus der Gruppe bestehend aus akutem Schmerz, chronischem Schmerz, neuropathischem Schmerz und visceralem Schmerz, besonders bevorzugt zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem erfindungsgemäßen *para-*Alkyl-substituierten N-(4-Hydroxy-3-methoxy-benzyl)-zimtsäureamids der vorstehend angegebenen allgemeinen Formel I bzw. Ia, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate; enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln. Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden *para-*Alkyl-substituierten N-(4-Hydroxy-3-methoxy-benzyl)-zimtsäureamide der vorstehend angegebenen allgemeinen Formeln I bzw. Ia in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen *para-*Alkyl-substituierten N-(4-Hydroxy-3-methoxy-benzyl)-zimtsäureamide der vorstehend angegebenen allgemeinen Formeln I bzw. Ia auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge des jeweiligen *para-*Alkyl-substituierten N-(4-Hydroxy-3-methoxy-benzyl)-zimtsäureamids der vorstehend angegebenen allgemeinen Formeln I bzw. Ia kann variieren und ist beispielsweise abhängig vom Gewicht oder Alter des Patienten sowie von der Applikationsart, der Indikation und dem Schweregrad der Erkrankung. Üblicherweise werden 0,005 bis 5000 mg/kg, vorzugsweise 0,05 bis 500 mg/kg Körpergewicht des Patienten wenigstens einer solchen Verbindung appliziert.

### Pharmakologische Methoden:

### 1.) Funktionelle Untersuchung am Vanilloid-Rezeptor 1 (VRI/TRPV1-Rezeptor)

Die agonistische bzw. antagonistische Wirkung der zu untersuchenden Substanzen am Vanilloid- Rezeptor 1 (VR1/TRPV1) der Spezies Mensch und Ratte kann mit folgendem Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Chinese Hamster Ovary Zellen (CHO K1 Zellen, European Collection of Cell Cultures (ECACC), England) werden stabil mit dem humanen bzw. dem Vanilloid-Rezeptor 1 (VR1)-Gen der Ratte transfiziert. Für funktionelle Untersuchungen werden diese Zellen auf Poly-D-Lysin-beschichtete, schwarze 96- Loch-Platten mit klarem Boden (BD Biosciences, Heidelberg, Deutschland) ausplattiert in einer Dichte von 25.000 Zellen/Loch. Über Nacht werden die Zellen bei 37 °C und 5 % CO₂ in Kulturmedium (Nutrient Mixture Ham's F12, Gibco Invitrogen GmbH, Karlsruhe, Deutschland) mit 10 Vol-% FBS (Fetal bovine serum, Gibco Invitrogen GmbH, Karlsruhe, Deutschland) und 18 µg/ml L-Prolin (Gibco Invitrogen GmbH, Karlsruhe, Deutschland) inkubiert. Am folgenden Tag werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen. Anschließend werden die Platten 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺- abhängige Fluoreszenz wird dabei vor und nach Zugabe von Substanzen gemessen (λex = 488 nm, λem = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden Testsubstanzen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Zur Bestimmung der EC₅₀-Werte werden die Substanzen in verschiedenen Konzentrationen zugegeben. Es werden Dreifach-Bestimmungen (n=3) durchgeführt und diese in mindestens 3 unabhängigen Experimenten wiederholt (N=4).

### 2. Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten hatten. Alle Verbindungen wurden in der Standarddosierung von 10 mg/kg getestet.

Im folgenden wird die Erfindung anhand eines Beispiels erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "Äquivalente" bedeutet Stoffmengenäquivalente, "RT" Raumtemperatur, "konz." konzentriert, "min" Minuten, "h" Stunden, "M" ist eine Konzentrationsangabe in mol/l, "aq." wäßrig.

**Weitere Abkürzungen:**

| | |
|---|---|
| BOP | 1 -Benzotriazolyl-tris-(dimethylamino)-phosphonium |
| DCM | Dichlormethan |
| DMF | N,N-Dimethylformamid |
| DIPE | Diisopropylether |
| EE | Essigsäureethylester |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den üblichen dem Fachmann bekannten Methoden synthetisiert.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR-Spektroskopie.

### Beispiel 4: para-tert-Butyl-trans-N-(4-hydroxy-3-methoxy-benzyl)-zimtsäure-amid

### a) Synthese von para-tert-Butyl-trans-zimtsäure

Malonsäure 19.20 g (0.185 mol) wurde in Pyridin (33 ml) gelöst und 15 min bei RT gerührt. Anschließend wurden 25.00 g (0.154 mmol) *para-*tert-Butylbenzaldehyd und 1.50 ml (0.020 mol) Piperidin zugegeben. Das Reaktionsgemisch wurde unter Rühren 8 Stunden auf 100 °C erhitzt. Nach Eingießen der Reaktionslösung in eine Mischung aus konz. Salzsäure und Eis wurde weitere 2 h bei RT gerührt. Der entstandene Niederschlag wurde abgesaugt. Durch Umkristallisieren der Niederschlags aus Methanol konnten 15.20 g (0.074 mol, 48 % der Theorie) *para-*tert-Butyl-*trans-*zimtsäure erhalten werden.

### b) Synthese von para-tert-Butyl-trans-N-(4-hydroxy-3-methoxy-benzyl)-zimtsäureamid

400 mg (1.96 mmol) *para-*tert-Butyl-*trans-*zimtsäure wurden bei 5 °C zusammen mit 290 mg (1.94 mmol) 4-Hydroxy-3-methoxybenzylamin Hydrochlorid und 0.48 ml (6.50 mmol) Triethylamin in DMF (10 ml) gelöst. Nach Zugabe einer Lösung von 870 mg (1.97 mmol) BOP in DCM (9 ml) wurde 16 h bei RT gerührt. Anschließend wurde die Reaktionslösung auf Wasser gegossen und 2x mit einer Mischung aus DIPE / EE (1:1) extrahiert. Die vereinigten organischen Phasen wurden mit 2M aq. Salzsäure und 3x mit 1 M aq. Natriumhydrogencarbonat-Lösung gewaschen und über Magnesiumsulfat getrocknet. Nach Entfernen der Lösungsmittel und Kristallisation aus DIPE wurden 384 mg (1.13 mmol, 58% der Theorie) *para-*tert-Butyl-*trans-*N-(4-hydroxy-3-methoxy-benzyl)-zimtsäureamid erhalten.

Die nachfolgenden Beispiele 1 bis 3 und 5 bis 9 wurden analog zu Beispiel 4 hergestellt.
1. *para*-Methyl-*trans-*N-(4-hydroxy-3-methoxy-benzyl)-zimtsäure-amid
2. *para*-Ethyl-*trans-*N-(4-hydroxy-3-methoxy-benzyl)-zimtsäure-amid
3. *para*-iso-Butyl-*trans-*N-(4-hydroxy-3-methoxy-benzyl)-zimtsäure-amid
5. *para*-Propyl-trans*-*N-(4-hydroxy-3-methoxy-benzyl)-zimtsäureamid
6. *para*-iso-Butyl-trans*-*N-(4-hydroxy-3-methoxy-benzyl)-zimtsäureamid
7. *para*-neo-Pentyl-trans-N-(4-hydroxy-3-methoxy-benzyl)-zimtsäureamid
8. *para*-Butyl-trans-N-(4-hydroxy-3-methoxy-benzyl)-zimtsäureamid
9. *para*-Octyl-trans-N-(4-hydroxy-3-methoxy-benzyl)-zimtsäureamid

### Pharmakologische Daten:

Die agonistische bzw. antagonistische Wirkung wurde am Vanilloid-Rezeptor 1 (VR1/TRPV1-Rezeptor) von Mensch und Ratte mit dem vorstehend beschriebenen FLIPR-Assay bestimmt.

Die untersuchten erfindungsgemäßen *para-*Alkyl-substituierten N-(4-Hydroxy-3-methoxy-benzyl)-zimtsäureamide zeigen eine ausgezeichnete agonistische Wirkung auf den Vanilloid-Rezeptor 1 (VR1lTRPV1-Rezeptor).

In der nachfolgenden Tabelle I sind die pharmakologischen Daten für *para-*Alkyl-substituierte N-(4-Hydroxy-3-methoxy-benzyl)-zimtsäureamide der allgemeinen Formel I wiedergegeben:

**Tabelle I.**

| **Verbindung gemäß Beispiel** | **VR1 (Ratte) (% Stimulation im Vgl. zu 10 µM CP)** | **VR1 (Mensch) (% Stimulation im Vgl. zu 10 µM CP)** | **VR1 (Ratte) EC₅₀ [nM]** | **VR1 (Mensch) EC₅₀ [nM]** |
|---|---|---|---|---|
| 1 | 92 | 56 | | |
| 2 | 84 | 71 | | |
| 3 | 97 | 71 | | |
| 4 | 106 | 115 | 0,129 ± 0,061 | 0,147 ± 0,043 |
| 5 | 119 | 102 | | |
| 6 | 141 | 103 | | |
| 7 | 142 | 102 | | |
| 8 | 132 | 110 | | |
| 9 | 126 | 97 | | |

Die erfindungsgemäßen *para-*Alkyl-substituierten N-(4-Hydroxy-3-methoxy-benzyl)-zimtsäureamide der vorstehend angegebenen allgemeinen Formel I führen ebenfalls zu einer ausgezeichneten Reduzierung des nozizeptiven Verhaltens im Writhing-Test an Mäusen wie in der nachfolgenden Tabelle II beschrieben.

**Tabelle II.**

| **Verbindung gemäß Beispiel** | **Dosis [mg/kg] (i.v.)** | **Reduzierung des nozizeptiven Verhaltens gegenüber Kontrollen [%]** |
|---|---|---|
| 2 | 0.1 | 54 |
| 3 | 0.1 | 52 |
| 4 | 0.1 | 63 |
| 5 | 0.316 | 55 |
| 6 | 0.1 | 70 |
| 9 | 0.1 | 47 |

## Patentansprüche

1. *para-*Alkyl-substituierte N-(4-Hydroxy-3-methoxy-benzyl)-zimtsäureamide der allgemeinen Formel I, 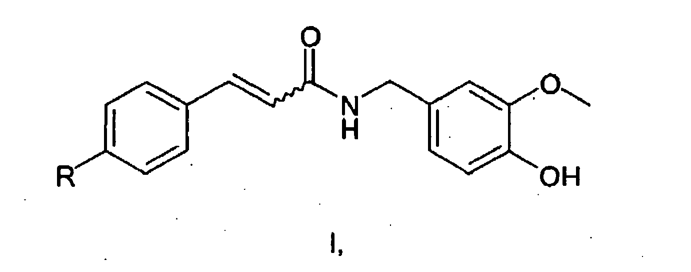 worin
R für einen linearen oder verzweigten Alkyl-Rest steht,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1 in Form ihrer *trans-*Isomere der allgemeinen Formel Ia, 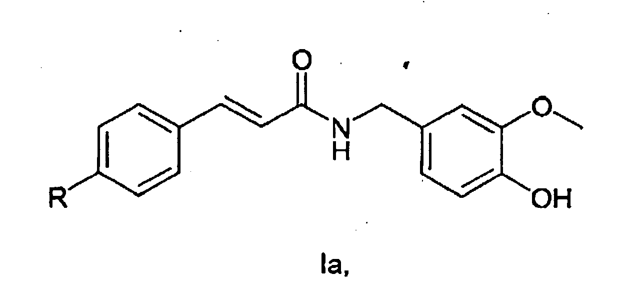 worin
R für einen linearen oder verzweigten Alkyl-Rest steht,
jeweils ggf. in Form eines ihrer reinen Stereoisomeren, insbesondere Enantiomeren, ihrer Racemate oder in Form einer Mischung von Enantiomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, dass** R für einen linearen oder verzweigten C₁₋₂₀-Alkyl-Rest steht.

4. Die Verbindungen gemäß einem oder mehreren der Ansprüche 1 bis 3 ausgewählt aus der Gruppe bestehend aus
[1] para-Methyl-*trans*-N-(4-hydroxy-3-methoxy-benzyl)-zimtsäureamid,
[2] para-Ethyl-*trans-*N-(4-hydroxy-3-methoxy-benzyl)-zimtsäureamid,
[3] para-iso-Propyl-trans-N-(4-hydroxy-3-methoxy-benzyl)-zimtsäureamid,
[4] *para*-tert-Butyl-*trans-*N-( 4-hydroxy-3-methoxy-benzyl)-zimtsäureamid,
[5] para-Propyl-trans-N-(4-hydroxy-3-methoxy-benzyl)-zimtsäureamid,
[6] para-iso-Butyl-trans-N-(4-hydroxy-3-methoxy-benzyl)-zimtsäureamid,
[7] para-neo-Pentyl-trans-N-(4-hydroxy-3-methoxy-benzyl)-zimtsäureamid,
[8] para-Butyl-trans-N-(4-hydroxy-3-methoxy-benzyl)-zimtsäureamid und
[9] para-Octyl-trans-N-(4-hydroxy-3-methoxy-benzyl)-zimtsäureamid;
jeweils ggf. in Form eines Salzes, oder jeweils ggf. in Form eines entsprechenden Solvates.

5. Verfahren zur Herstellung von Verbindungen gemäß einem oder mehreren der Ansprüche 1 bis 4, **dadurch gekennzeichnet, dass** wenigstens ein Aldehyd der allgemeinen Formel II, 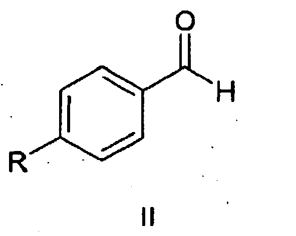 worin R die Bedeutung gemäß einem oder mehreren der Ansprüche 1 bis 4 hat, mit Malonsäure, ggf. in einem Reaktionsmedium, in Gegenwart wenigstens einer Base, umgesetzt wird, die so erhaltene *para-*Alkyl substituierte Zimtsäure der allgemeinen Formel III, 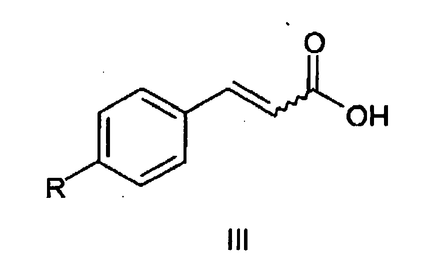 worin R die vorstehend genannte Bedeutung hat, ggf. in Form eines entsprechenden Salzes, ggf. isoliert und ggf. gereinigt wird, und durch Umsetzung mit 4-Hydroxy-3-methoxybenzylamin, ggf. in Form eines entsprechenden Salzes, in einem Reaktionsmedium, ggf. in Gegenwart wenigstens einer Base, ggf. in Gegenwart wenigstens eines geeigneten Kopplungsmittels, in eine entsprechende Verbindung der allgemeinen Formel I, 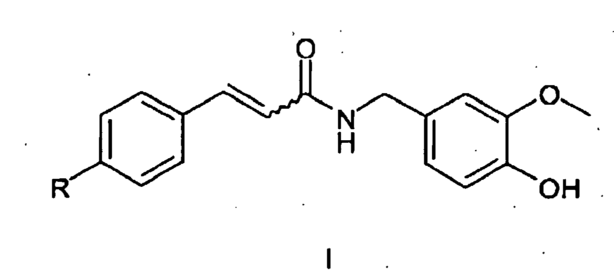 ggf. in Form eines entsprechenden Salzes überführt wird, worin R die vorstehend genannte Bedeutung hat, und diese ggf. gereinigt und ggf. isoliert wird.

6. Arzneimittel enthaltend wenigstens eine Verbindung gemäß einem oder mehreren der Ansprüche 1 bis 4 und ggf. einen oder mehrere physiologisch verträgliche Hilfsstoffe.

7. Arzneimittel gemäß Anspruch 6 zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen, Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt Gedächtnisstörungen; Angstzuständen; Epilepsie; Atemwegserkrankungen, vorzugsweise ausgewählt aus der Gruppe bestehend aus Asthma und Lungenentzündungen; Husten; Haminkontinenz; einer überaktiven Blase (overactive bladder, OAB); Diarrhöe; Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise Toferanzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmißbrauch; Entzugserscheinungen bei Älkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

8. Verwendung wenigstens einer Verbindung gemäß einem oder mehreren der Ansprüche 1 bis 4 zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt Gedächtnisstörungen; Angstzuständen; Epilepsie; Atemwegserkrankungen, vorzugsweise ausgewählt aus der Gruppe bestehend aus Asthma und Lungenentzündungen; Husten; Haminkontinenz; einer überaktiven Blase (overactive bladder, OAB); Diarrhöe; Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmißbrauch; Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie, vorzugsweise zur Herstellung eines Arzneimittels zur Prophylaxe und/oder Behandlung von Harninkontinenz oder zur Herstellung eines Arzneimittels zur Prophylaxe und/oder Behandlung von Schmerz, bevorzugt von Schmerz ausgewählt aus der Gruppe bestehend aus akutem Schmerz, chronischem Schmerz, neuropathischem Schmerz und visceralem Schmerz, besonders bevorzugt zur Herstellung eines Arzneimittels zur Prophylaxe und/oder Behandlung von Schmerzen, bevorzugt von Schmerzen ausgewählt aus der Gruppe bestehend aus akuten Schmerzen, chronischen Schmerzen, neuropathischen Schmerzen und visceralen Schmerzen.

## Claims

1. *para-*alkyl-substituted N-(4-hydroxy-3-methoxybenzyl)-cinnamic acid amides of the general formula I 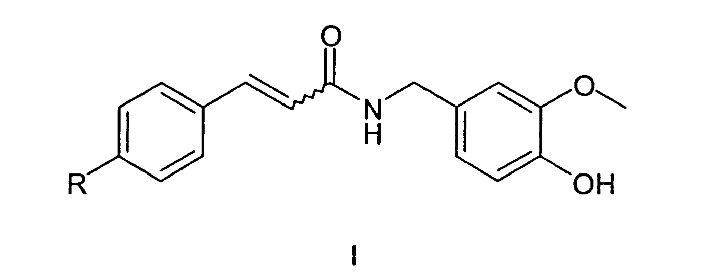 wherein
R stands for a linear or branched alkyl radical,
respectively possibly in the form of one of their pure stereoisomers, in particular enantiomers or diastereomers, their racemates or in the form of a mixture of stereoisomers, particularly of enantiomers and/or diastereomers, in any desired mixing ratio, or respectively in the form of appropriate salts, or respectively in the form of appropriate solvates.

2. Compounds according to claim 1 in the form of their *trans-*isomers of the general formula Ia 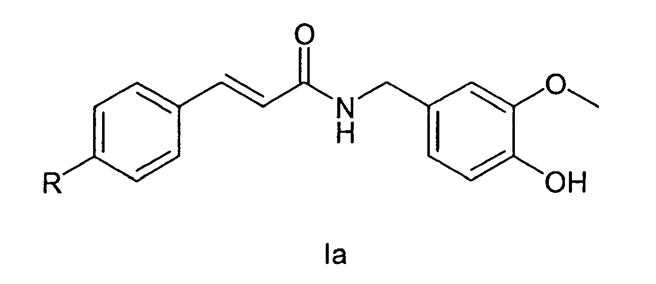 wherein
R stands for a linear or branched alkyl radical,
respectively possibly in the form of one of their pure stereoisomers, in particular enantiomers, their racemates or in the form of a mixture of enantiomers, in any desired mixing ratio, or respectively in the form of appropriate salts, or respectively in the form of appropriate solvates.

3. Compounds according to claim 1 or claim 2, **characterised in that** R stands for a linear or branched C₁₋₂₀ alkyl radical.

4. The compounds according to one or more of claims 1 to 3 selected from the group comprising
[1] *para*-methyl-*trans-*N-(4-hydroxy-3-methoxybenzyl)-cinnamic acid amide,
[2] *para-*ethyl-*trans-*N-(4-hydroxy-3-methoxybenzyl)-cinnamic acid amide,
[3] *para-*isopropyl-*trans-*N-(4-hydroxy-3-methoxybenzyl)-cinnamic acid amide,
[4] *para-*tert-butyl-*trans-*N-(4-hydroxy-3-methoxybenzyl)-cinnamic acid amide,
[5] *para-*propyl-*trans-*N-(4-hydroxy-3-methoxybenzyl)-cinnamic acid amide,
[6] *para-*isobutyl-*trans-*N-(4-hydroxy-3-methoxybenzyl)-cinnamic acid amide,
[7] *para-*neopentyl-*trans-*N-(4-hydroxy-3-methoxybenzyl)-cinnamic acid amide,
[8] *para-*butyl-*trans-*N-(4-hydroxy-3-methoxybenzyl)-cinnamic acid amide; and
[9] *para-*octyl-*trans-*N-(4-hydroxy-3-methoxybenzyl)-cinnamic acid amide;
respectively possibly in the form of a salt or respectively possibly in the form of an appropriate solvate.

5. Process for the preparation of compounds according to one or more of claims 1 to 4, **characterised in that** at least one aldehyde of the general formula II 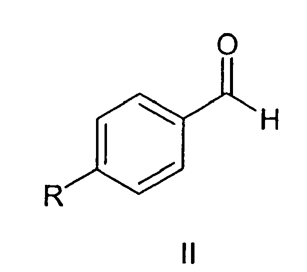 wherein R has the meaning stated in one or more of claims 1 to 4, is caused to react with malonic acid, possibly in a reaction medium, in the presence of at least one base, the resulting *para-*alkyl-substituted cinnamic acid of the general formula III 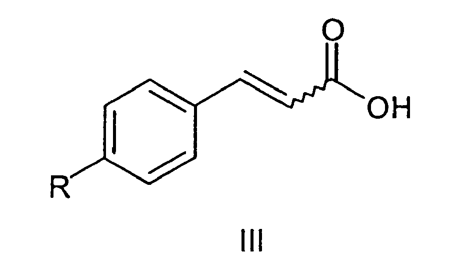 wherein R has the meaning stated above, possibly in the form of an appropriate salt, is possibly isolated and possibly purified,
and is caused to react with 4-hydroxy-3-methoxybenzylamine, possibly in the form of an appropriate salt, in a reaction medium, possibly in the presence of at least one base, possibly in the presence of at least one suitable coupling agent, to form a corresponding compound of the general formula I 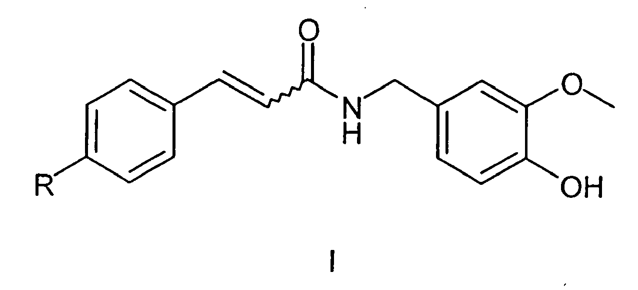 possibly in the form of an appropriate salt, wherein R has the meaning stated above, and this compound is possibly purified and possibly isolated.

6. Medicinal drug comprising at least one compound according to one or more of claims 1 to 4 and possibly one or more physiologically acceptable adjuvants.

7. Medicinal drug according to claim 6 for the treatment and/or prophylaxis of pain, preferably pain selected from the group comprising acute pain, chronic pain, neuropathic pain and visceral pain; arthralgia; migraine; depression; nervous disorders; neurotrauma; neurodegenerative diseases, preferably selected from the group comprising multiple sclerosis, Alzheimer's disease, Parkinson's disease and Huntington's disease; cognitive disorders, preferably cognitive deficiencies, particularly preferred memory disorders; anxiety; epilepsy; respiratory tract diseases, preferably selected from the group comprising asthma and pneumonia; coughing; urinary incontinence; overactive bladder (OAB); diarrhoea; gastric ulcers; irritable bowel syndrome; strokes; irritations of the eyes; skin irritations; neurotic skin diseases; inflammatory diseases, preferably inflammation of the colon; pruritus; eating disorders, preferably selected from the group comprising bulimia, cachexia, anorexia, and obesity; medication dependency; medication abuse; withdrawal symptoms in the case of medication dependency; development of tolerance to medications, preferably to natural or synthetic opioids; drug dependency; drug abuse; withdrawal symptoms in the case of drug dependency; alcohol dependency; alcohol abuse; withdrawal symptoms in the case of alcohol dependency; for diuresis; for antinatriuresis; for influencing the cardiovascular system; for increasing vigilance; for increasing libido; for modulating motor activity, or for local anaesthesia.

8. Use of at least one compound according to one or more of claims 1 to 4 for the preparation of a medication for the treatment and/or prophylaxis of pain, preferably pain selected from the group comprising acute pain, chronic pain, neuropathic pain and visceral pain; arthralgia; migraine; depression; nervous disorders; neurotrauma; neurodegenerative diseases, preferably selected from the group comprising multiple sclerosis, Alzheimer's disease, Parkinson's disease and Huntington's disease; cognitive disorders, preferably cognitive deficiencies, particularly preferred memory disorders; anxiety; epilepsy; respiratory tract diseases, preferably selected from the group comprising asthma and pneumonia; coughing; urinary incontinence; overactive bladder (OAB); diarrhoea; gastric ulcers; irritable bowel syndrome; strokes; irritations of the eyes; skin irritations; neurotic skin diseases; inflammatory diseases, preferably inflammation of the colon; pruritus; eating disorders, preferably selected from the group comprising bulimia, cachexia, anorexia and obesity; medication dependency; medication abuse; withdrawal symptoms in the case of medication dependency; development of tolerance to medications, preferably to natural or synthetic opioids; drug dependency; drug abuse; withdrawal symptoms in the case of drug dependency; alcohol dependency; alcohol abuse; withdrawal symptoms in the case of alcohol dependency; for diuresis; for antinatriuresis; for influencing the cardiovascular system; for increasing vigilance; for increasing libido; for modulating motor activity or for local anaesthesia, preferably for the preparation of a medicinal drug for the prophylaxis and/or treatment of urinary incontinence, or for the preparation of a medicinal drug for the prophylaxis and/or treatment of pain, preferably pain selected from the group comprising acute pain, chronic pain, neuropathic pain and visceral pain, particularly preferred for the preparation of a medicinal drug for the prophylaxis and/or treatment of pain, preferably pain selected from the group comprising acute pain, chronic pain, neuropathic pain and visceral pain.

## Revendications

1. Amides de l'acide N-(4-hydroxy-3-méthoxybenzyl)-cinnamique substitués par alkyle en position para de formule générale I, 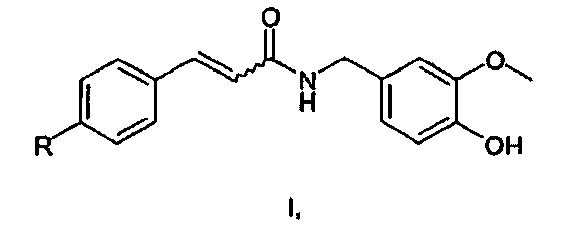 où
R représente un radical alkyle linéaire ou ramifié, le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

2. Composés selon la revendication 1 sous forme de leurs isomères trans de formule générale Ia 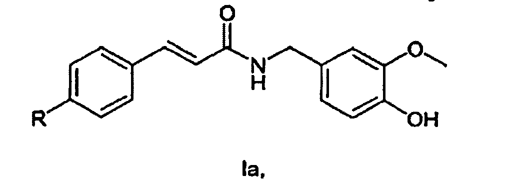 où
R représente un radical alkyle linéaire ou ramifié, le cas échéant à chaque fois sous forme d'un de leurs stéréo-isomères purs, en particulier leurs énantiomères, leurs racémates ou sous forme d'un mélange d'énantiomères, dans un rapport de mélange quelconque ou à chaque fois sous forme de sels correspondants ou à chaque fois sous forme de solvates correspondants.

3. Composés selon la revendication 1 ou 2,
**caractérisés en ce que** R représente un radical C₁₋₂₀-alkyle linéaire ou ramifié,

4. Composés selon l'une ou plusieurs des revendications 1 à 3 choisis dans le groupe constitué par
1. l'amide de l'acide para-méthyl-trans-N-(4-hydroxy-3-méthoxybenzyl)-cinnamique,
2. l'amide de l'acide para-éthyl-trans-N-(4-hydroxy-3-méthoxybenzyl)-cinnamique,
3. l'amide de l'acide para-iso-propyl-trans-N-(4-hydroxy-3-méthoxybenzyl)-cinnamique,
4. l'amide de l'acide para-tert-butyl-trans-N-(4-hydroxy-3-méthoxybenzyl)-cinnamique,
5. l'amide de l'acide para-propyl-trans-N-(4-hydroxy-3-méthoxybenzyl)-cinnamique,
6. l'amide de l'acide para-iso-butyl-trans-N-(4-hydroxy-3-méthoxybenzyl)-cinnamique,
7. l'amide de l'acide para-néo-pentyl-trans-N-(4-hydroxy-3-méthoxybenzyl)-cinnamique,
8. l'amide de l'acide para-butyl-trans-N-(4-hydroxy-3-méthoxybenzyl)-cinnamique et
9. l'amide de l'acide para-octyl-trans-N-(4-hydroxy-3-méthoxybenzyl)-cinnamique ;
à chaque fois le cas échéant sous forme d'un sel ou à chaque fois le cas échéant sous forme d'un solvate correspondant.

5. Procédé pour la préparation de composés selon l'une ou plusieurs des revendications 1 à 4, **caractérisé en ce qu'**au moins un aldéhyde de formule générale II, 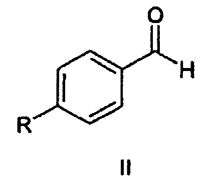 où R a la signification selon l'une ou plusieurs des revendications 1 à 4, est transformé avec de l'acide malonique, le cas échéant dans un milieu réactionnel, en présence d'au moins une base, l'acide cinnamique substitué par alkyle en position para de formule générale III ainsi obtenu 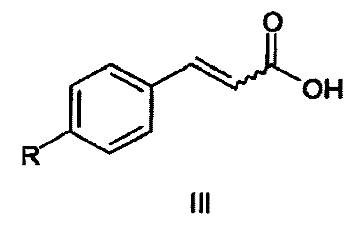 où R à la signification susmentionnée, le cas échéant sous forme d'un sel correspondant et le cas échéant purifié et le cas échéant isolé, et transformé par transformation avec de la 4-hydroxy-3-méthoxybenzylamine, le cas échéant sous forme d'un sel correspondant, dans un milieu réactionnel, le cas échéant en présence d'au moins une base,
le cas échéant en présence d'au moins un agent de couplage approprié, en un composé correspondant de formule générale I, 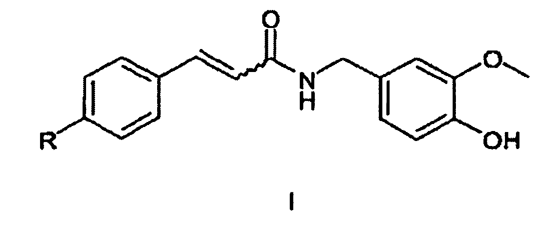 le cas échéant sous forme d'un sel correspondant, où R a la signification susmentionnée, et celui-ci est le cas échéant purifié et le cas échéant isolé.

6. Médicament contenant au moins un composé selon l'une ou plusieurs des revendications 1 à 4 et le cas échéant un ou plusieurs adjuvants physiologiquement acceptables.

7. Médicament selon la revendication 6 destiné au traitement et/ou à la prophylaxie de la douleur, de préférence de la douleur choisie dans le groupe constitué par la douleur aiguë, la douleur chronique, la douleur neuropathique et la douleur viscérale ; la douleur articulaire ; la migraine ; les dépressions ; les maladies nerveuses ; les lésions nerveuses ; les maladies de neurodégénérescence, de préférence choisies dans le groupe constitué par la sclérose en plaques, la maladie d'Alzheimer, la maladie de Parkinson et la maladie d'Huntington ; les maladies cognitives, de préférence les états de carence cognitive, de manière particulièrement préférée les troubles de la mémoire ; les états d'anxiété ; l'épilepsie ; les maladies des voies respiratoires, de préférence choisies dans le groupe constitué par l'asthme et les inflammations pulmonaires ; la toux ; l'incontinence urinaire ; une vessie hyperactive (overactive bladder, OAB) ; la diarrhée ; les ulcères d'estomac ; le syndrome de l'intestin irritable ; les attaques ; les irritations oculaires ; les irritations de la peau ; les maladies neurotiques de la peau ; les maladies inflammatoires, de préférence les inflammations de l'intestin ; le prurit ; les troubles de l'absorption des aliments, de préférence choisis dans le groupe constitué par la boulimie, la cachexie, l'anorexie et l'obésité ; la dépendance aux médicaments ; l'usage abusif de médicaments ; les phénomènes de sevrage lors de la dépendance aux médicaments ; le développement d'une tolérance aux médicaments, de préférence le développement d'une tolérance aux opioïdes naturels ou synthétiques ; la dépendance aux drogues ; l'usage abusif de drogues ; les phénomènes de sevrage lors de la dépendance aux drogues ; la dépendance à l'alcool ; l'usage abusif d'alcool ; les phénomènes de sevrage lors de la dépendance à l'alcool ; à la diurèse ; à l'antinatriurèse ; à l'influence du système cardiovasculaire ; à l'augmentation de la vigilance ; à l'augmentation de la libido ; à la modulation de l'activité de la mobilité ou à l'anesthésie locale.

8. Utilisation d'au moins un composé selon l'une ou plusieurs des revendications 1 à 4 pour la préparation d'un médicament destiné au traitement et/ou à la prophylaxie de la douleur, de préférence de la douleur choisie dans le groupe constitué par la douleur aiguë, la douleur chronique, la douleur neuropathique et la douleur viscérale ; la douleur articulaire ; la migraine ; les dépressions ; les maladies nerveuses ; les lésions nerveuses ; les maladies de neurodégénérescence, de préférence choisies dans le groupe constitué par la sclérose en plaques, la maladie d'Alzheimer, la maladie de Parkinson et la maladie d'Huntington ; les maladies cognitives, de préférence les états de carence cognitive, de manière particulièrement préférée les troubles de la mémoire ; les états d'anxiété ; l'épilepsie ; les maladies des voies respiratoires, de préférence choisies dans le groupe constitué par l'asthme et les inflammations pulmonaires ; la toux ; l'incontinence urinaire ; une vessie hyperactive (overactive bladder, OAB) ; la diarrhée ; les ulcères d'estomac ; le syndrome de l'intestin irritable ; les attaques ; les irritations oculaires ; les irritations de la peau ; les maladies neurotiques de la peau ; les maladies inflammatoires, de préférence les inflammations de l'intestin ; le prurit ; les troubles de l'absorption des aliments, de préférence choisis dans le groupe constitué par la boulimie, la cachexie, l'anorexie et l'obésité ; la dépendance aux médicaments ; l'usage abusif de médicaments ; les phénomènes de sevrage lors de la dépendance aux médicaments ; le développement d'une tolérance aux médicaments, de préférence aux opioïdes naturels ou synthétiques ; la dépendance aux drogues ; l'usage abusif de drogues ; les phénomènes de sevrage lors de la dépendance aux drogues ; la dépendance à l'alcool ; l'usage abusif d'alcool ; les phénomènes de sevrage lors de la dépendance à l'alcool ; à la diurèse ; à l'antinatriurèse ; à l'influence du système cardiovasculaire ; à l'augmentation de la vigilance ; à l'augmentation de la libido ; à la modulation de l'activité de la mobilité ou à l'anesthésie locale, de préférence pour la préparation d'un médicament destiné à la prophylaxie et/ou au traitement de l'incontinence urinaire ou pour la préparation d'un médicament destiné à la prophylaxie et/ou au traitement de la douleur, de préférence de la douleur choisie dans le groupe constitué par la douleur aiguë, la douleur chronique, la douleur neuropathique et la douleur viscérale, de manière particulièrement préférée pour la préparation d'un médicament destiné à la prophylaxie et/ou au traitement de douleurs, de préférence de douleurs choisies dans le groupe constitué par les douleurs aiguës, les douleurs chroniques, les douleurs neuropathiques et les douleurs viscérales.
